# EUROPEAN PATENT APPLICATION

(11) **EP 4 789 724 A2**
(43) Date of publication of application: **12.08.2026**
(21) Application number: 26185820.3
(22) Date of filing: 22.09.2023
(51) Int. Cl.: A61P 35/00

(54) **ANTI-4-1BB NANOBODY 137-7, PREPARATION THEREFOR, AND USE THEREOF**

(30) Priority: 22.09.2022 CN 202211160125
(62) Document number(s) of the earlier application(s) in accordance with Art. 76 EPC: 23867649.8 / 4 592 317
(71) Applicant: ABlink Biotech Co., Ltd., Chengdu, Sichuan 610093 (CN)
(72) Inventor: LI, Xinlei, Chengdu, Sichuan, 610093 (CN); ZENG, Xin, Chengdu, Sichuan, 610093 (CN); LIU, Jianghai, Chengdu, Sichuan, 610093 (CN); LI, Lingfang, Chengdu, Sichuan, 610093 (CN); DU, Jianhua, Chengdu, Sichuan, 610093 (CN); ZHU, Di, Chengdu, Sichuan, 610093 (CN)
(74) Representative: Cabinet Beau de Loménie

(57) **Abstract**

The present application relates to the field of biomedicine technology, and particularly to an anti-4-1BB nanobody and its preparation and application.

## Description

### TECHNICAL FIELD

The present application relates to the field of biomedicine technology, and particularly to an anti-4-1BB nanobody and its preparation and application.

### BACKGROUND

Effective stimulation of T cell immune function requires a co-stimulatory process and a cytokine environment to promote cell cycle progression, survival, and proliferation. 4-1BB (CD137) is one of the co-stimulatory members of the tumor necrosis factor receptor superfamily (TNFRSF). It is an inducible T cell surface receptor that is not only highly expressed on activated T cells, but also continues to retain on T cells. The team of Pollok K E,Kim S H demonstrated that, anti-CD3 antibodies or concanavalin (concanavalin A, Con A) stimulate high expression of 4-1 BB on splenic T cells. The natural ligand of 4-1 BB, 4-1BBL, exists on activated antigen-presenting cells (APCs). When 4-1BB binds to its natural ligand or agonistic monoclonal antibody (MoAb), it delivers a unique co-stimulatory signal playing a role in T cell response in vivo and in vitro, including stimulating T cell proliferation, up-regulating survival-related genes, and producing Th1 cytokines such as IL-2, IFN-y, and TNF-α. Among them, IL-2 is one of the main positive growth factors of T cells. High level of IL-2 secreted by CD8+ T cells plays a critical role in inducing cell cycle progression, producing IFN-γ and other cytokines, and inducing memory CD8+ T cells. Furthermore, 4-1BB-mediated signaling plays a critical role in preventing activation-induced cell death, promoting rejection of cardiac allografts and skin grafts, increasing the cytolytic capacity of CD8+ T cells, and eradicating tumors.

Nanobodies (Nb) are the variable regions of heavy chain antibodies (IgG2 and IgG3) in camelids and are known as the smallest antigen-binding fragments existing in nature. Compared with traditional full-length monoclonal antibodies (mAb, approximately 150KD), Nb has a small molecular weight (12-15KD), simpler structure, low immunogenicity, high tissue penetration, high stability, high solubility and low aggregation and ease of cloning. In addition, Nb production costis significantly reduced as compared to mAb counterpart, making it accessible to most cancer patients.

Currently, 4-1BB monoclonal antibodies still face many challenges, including: 1) large molecular size (160-170kD), which limits their ability to penetrate solid tumors and the blood-brain barrier, and 2) in vitro eukaryotic expression and cell screening processes involve large amount of work, resulting in high production costs.

### SUMMARY

In view of this, the present invention provides an anti-4-1BB Nanobody and a preparation method and use thereof. The anti-4-1BB nanobody has the advantages of small molecular weight, simpler structure, low immunogenicity, high tissue permeability, high stability, high solubility and low aggregation, and easy cloning, and can be used for increasing the cytolytic ability of CD8+ T cells and treatment or prevention of tumors.

In order to achieve the above-mentioned object of the invention, the present invention provides the following technical solutions:
The invention provides an anti-4-1BB antibody and a variant thereof, or an antigen-binding fragment thereof. The anti-4-1BB antibody comprises three complementary determining regions CDR1, CDR2 and CDR3 of VHHs respectively named as 137-1, 137-7, 137-12, 137-16, 137-18, 137-36 and 137-39 (as shown in the table below),

| **SEQ ID NOs:** | **Unique identifier** | **VHH amino acid sequence** | **source** |
|---|---|---|---|
| 1 | 137-1 | QVQLVESGGGLVQPGGSLRLSCVASG FVFSTVTMGWGRQVPGKEFEWVSGVS SEGITDYADSVKGRFTISRDNAKNTVY LEMNSLKSEDTAEYYCLIGGRWSFGT WGQGTQVTVSS | Phage ELISA Positive |
| 2 | 137-7 | QVQLVESGGGLVQPGGSLRLSCAASG FTLDSYAIAWFRQAPGKEREGVSCITSS GGSTNYADSVKGRFTISRDGAKNTVYL QLNSLKPEDTAIYYCAAFHRATCVILG EYDYWGQGTQVTVSS | NGS Top7 |
| 3 | 137-12 | QVQLVESGGGLVHPGGSLRLSCAASG FTLDDSAIGWFRQAPGKEREGVSCRSR SGYMTKYADSVKGRFTISRYNANNTMY LQMNSLKPEDTAVYYCATTRRGYCSG YETKGVDYWGQGIQVTVSS | NGS Top12 |
| 4 | 137-16 | EVQLVESGGGLVQAGASLTLSCVISG RTFSSYAMGWYRQAPGKERAFVGTIT WNNDIIYSDSVKGRFTISTDDGKNTMY LQMNNLKPEDTAVYYCAAYSVPYGDF CTLNYDLMDYWGKGTLVTVSS | NGS Top16 |
| 5 | 137-18 | QVQLVESGGGLVHPGGSLRLSCAASG FIFSLYTMSWVRQAPGKGLEWVSDINS GGDSTDYADSVKGRFTISRDNAKSTLY LQMNSLKPEDTAVYYCAKDYPLGQRE DYWGQGTQVTVSS | NGS Top18 |
| 6 | 137-36 | QVQLVESGGGLVQTGGSLTLSCVASG RRFEAYTIGWLRQAPGDLREGVGCIFG GTTFTGYDDSVKGRFTISRDTAKNTVY LRMNNLTSEDTATYYCAAEGRPGQIK GQHGCLTPSWYNYKGQGTQVTVSS | NGS Top36 |
| 7 | 137-39 | QVQLVESGGGLVHVGESLTLSCVISG GSSNLYGIGWYRQAPGQERKLVAVLH NEHTTKYLDSVKGRFFVSGDNAKNTLY LHMNNVKVEDTAVYYCNSASGTLAGT GYFASVGPWGQGTLVTVSS | NGS Top39 |
| 8 | CDR1 | TVTMG | 137-1 |
| 9 | CDR2 | GVSSEGITDYADSVKG | 137-1 |
| 10 | CDR3 | GGRWSFGT | 137-1 |
| 11 | CDR1 | SYAIA | 137-7 |
| 12 | CDR2 | CITSSGGSTNYADSVKG | 137-7 |
| 13 | CDR3 | FHRATCVILGEYDY | 137-7 |
| 14 | CDR1 | DSAIG | 137-12 |
| 15 | CDR2 | CRSRSGYMTKYADSVKG | 137-12 |
| 16 | CDR3 | TRRGYCSGYETKGVDY | 137-12 |
| 17 | CDR1 | SYAMG | 137-16 |
| 18 | CDR2 | TITWNNDIIYSDSVKG | 137-16 |
| 19 | CDR3 | YSVPYGDFCTLNYDLMDY | 137-16 |
| 20 | CDR1 | LYTMS | 137-18 |
| 21 | CDR2 | DINSGGDSTDYADSVKG | 137-18 |
| 22 | CDR3 | DYPLGQREDY | 137-18 |
| 23 | CDR1 | AYTIG | 137-36 |
| 24 | CDR2 | CIFGGTTFTGYDDSVKG | 137-36 |
| 25 | CDR3 | EGRPGQIKGQHGCLTPSWYNY | 137-36 |
| 26 | CDR1 | LYGIG | 137-39 |
| 27 | CDR2 | VLHNEHTTKYLDSVKG | 137-39 |
| 28 | CDR3 | ASGTLAGTGYFASVGP | 137-39 |

Exemplarily, for the antibody and a variant thereof described herein, or an antigen-binding fragment thereof, the variant is a humanized variant or a homology variant (e.g., having at least 80%, 81%, 82%, 83%, 84% , 85%, 86%, 87%, 88%, 89%, 90%, 91%, 92%, 93%, 94%, 95%, 96%, 97%, 98%, 99%, 99.5%, or 99.9 % identity).

Further, for the antibody and a variant thereof described herein, or an antigen-binding fragment thereof, it is selected from camel Ig, Ig NAR, Fab fragment, Fab' fragment, F(ab)'₂ fragment, F(ab)'₃ fragment, Fv, scFv, bi-scFv, (scFv)₂, mini-antibody,double-chain antibody, three-chain antibody, four-chain antibody, disulfide-stabilized Fv protein, and single domain antibody (sdAb, nanoantibody), camel antibody, bispecific antibody or trispecific antibody.

The present invention also provides a fusion protein comprising the antibody and a variant thereof, or an antigen-binding fragment thereof described herein.

Exemplarily, for the fusion protein described herein, it also comprises a tag sequence (such as Poly-His, Hemagglutinin, c-Myc, GST, Flag-tag, etc.) or an IgG1-Fc protein sequence, or an additional epitope (such as targeting 4-1BB or targeting or other epitopes other than 4-1BB) or an additional antibody active fragment (such as an antibody or an antibody active fragment targeting other epitopes of 4-1BB or the same epitope, or a ligand capable of binding to 4-1BB).

The present invention also provides an antibody-drug conjugate, which comprises the antibody and a variant thereof, or an antigen-binding fragment thereof described herein.

For the antibody-drug conjugate described herein, the drug is selected from the followings: radioactive label, ³²P, ³⁵S, fluorescent dye, electron-dense reagent, enzyme, biotin, streptavidin, digoxigenin, hapten, immunogenic protein, nucleic acid molecule with sequence complementary to a target, or any combination of the foregoing; or immune-modulatory compound, anticancer agent, antiviral agent, antibacterial agent, antifungal agent and antiparasitic agent, or any combination of the foregoing.

The present invention also provides an isolated polynucleotide expressing an antibody or an antigen-binding fragment thereof, wherein the polynucleotide is capable of expressing the antibody and a variant thereof, or an antigen-binding fragment thereof described herein; the polynucleotide is capable of expressing fusion protein described herein; the polynucleotide is capable of expressing the antibody-drug conjugate described herein.

The invention also provides a vector comprising the polynucleotide described herein, preferably the vector is a plasmid vector.

The invention also provides a host cell comprising the polynucleotide or the vector described herein. Preferably, the host cell is a eukaryotic cell.

The present invention also provides a pharmaceutical composition comprising the antibody and a variant thereof, or an antigen-binding fragment thereof described herein, the fusion protein described herein, the antibody-drug conjugate described herein, and optionally a pharmaceutically acceptable carrier.

The present invention also provides use of the antibody and a variant thereof, or an antigen-binding fragment thereof described herein, the fusion protein described herein, and the antibody-drug conjugate described herein in the preparation of a medicament for treating and/or preventing a disease associated with 4-1BB.

The present invention also provides a method for treating and/or preventing a disease associated with 4-1BB and the related symptoms, wherein the method comprises administrating an effective amount of the antibody and a variant thereof, or an antigen-binding fragment thereof described herein, the fusion protein described herein, the antibody-drug conjugate described herein, or the pharmaceutical composition described herein to a subject.

The present invention also provides a method for detecting whether a sample comprises T cells highly expressing 4-1BB, wherein the method comprises a step of contacting the sample with the antibody and a variant thereof, or an antigen-binding fragment thereof described herein, and the fusion protein described herein, optionally the detection can be for a diagnostic purpose or non-diagnostic purpose.

The invention also provides a detection product comprising the antibody and a variant thereof, or an antigen-binding fragment thereof described herein.

Exemplarily, for the detection product described herein, the product is one or more selected from a detection reagent, a kit, a chip or a test paper.

In order to achieve the above-mentioned object of the invention, the present invention further provides the following technical solutions:
A first aspect of the present invention provides an anti-4-1BB nanobody, wherein the heavy chain variable region of the anti-4-1BB nanobody consists of a framework region FR and a complementary determining region CDR, and wherein the complementary determining region CDR comprises at least one of the following groups:
(1) CDR1, CDR2 and CDR3 set forth in SEQ ID NO: 1;
(2) CDR1, CDR2 and CDR3 set forth in SEQ ID NO: 2;
(3) CDR1, CDR2 and CDR3 set forth in SEQ ID NO: 3;
(4) CDR1, CDR2 and CDR3 set forth in SEQ ID NO: 4;
(5) CDR1, CDR2 and CDR3 set forth in SEQ ID NO: 5;
(6) CDR1, CDR2 and CDR3 set forth in SEQ ID NO: 6; and
(7) CDR1, CDR2 and CDR3 set forth in SEQ ID NO:7.

Nanobodies (Nb) are the variable regions of heavy chain antibodies (IgG2 and IgG3) in camelids and are known as the smallest antigen-binding fragments existing in nature. Compared with traditional full-length monoclonal antibodies (mAb, approximately 150KD), Nb has a small molecular weight (12-15KD), simpler structure, low immunogenicity, high tissue penetration, high stability, high solubility and low aggregation and ease of cloning. In addition, Nb production cost is significantly reduced as compared to mAb counterpart, making it accessible to most cancer patients. In September 2018, the European Medicines Agency approved the first nanobody drug caplacizumab (trade name: Cablivi), which is mainly used to treat acquired thrombotic thrombocytopenic purpura (aTTP) in adults. Therefore, Nbs are expected to provide a wide range of application scenarios in cancer treatment and diagnosis.

In this study, recombinant 4-1BB antigen was used to immunize camels to construct a VHH phage library. After biopanning, 7 functional anti-4-1BB Nbs (such as the antibodies set forth in SEQ ID Nos: 1-7) were obtained.

In the embodiment provided by the present invention, the framework region FR comprises at least one of the following groups (1)-(7):
(1) FR1, FR2, FR3 and FR4 set forth in SEQ ID NO: 1;
(2) FR1, FR2, FR3 and FR4 set forth in SEQ ID NO: 2;
(3) FR1, FR2, FR3 and FR4 set forth in SEQ ID NO: 3;
(4) FR1, FR2, FR3 and FR4 set forth in SEQ ID NO: 4;
(5) FR1, FR2, FR3 and FR4 set forth in SEQ ID NO: 5;
(6) FR1, FR2, FR3 and FR4 set forth in SEQ ID NO: 6; and
(7) FR1, FR2, FR3 and FR4 set forth in SEQ ID NO: 7.

However, the framework region FR of the present invention is not limited to the above sequences, and any sequence that can achieve its function is within the protection scope of the present invention.

A second aspect of the present invention provides a polynucleotide encoding the above anti-4-1BB nanobody.

A third aspect of the present invention provides a recombinant expression vector, which comprises the above polynucleotide.

In the embodiments provided by the present invention, the expression vector comprises a prokaryotic expression vector or a eukaryotic expression vector.

A fourth aspect of the present invention provides a recombinant host cell, which comprises the above polynucleotide, or comprises the above recombinant expression vector.

In the embodiments provided by the present invention, the host cells include prokaryotic cells or eukaryotic cells.

In a particular embodiment provided by the present invention, the host cell is selected from *Escherichiacoli* or yeast cell.

In a particular embodiment provided by the present invention, the host cell is selected from HEK293T cell, HEK293F cell, Expi293F cell or CHO cell.

A fifth aspect of the present invention provides a method for preparing the aboved anti-4-1BB nanobody, wherein the method comprises the following steps:
inserting the polynucleotide encoding the above anti-4-1BB nanobody into an expression vector to obtain a recombinant expression vector;
transferring the recombinant expression vector intoa host cell to obtain the recombinant host cell;
culturing the recombinant host cell to obtain a culture; and
purifying the culture to obtain anti-4-1BB nanobodies.

In the embodiments provided by the present invention, purification is performed by using Protein A agarose purification resin.

A sixth aspect of the present invention provides a bispecific antibody, wherein the bispecific antibody comprises the above anti-4-1BB nanobody and a second antibody.

In the embodiments provided by the present invention, the second antibody includes but are not limited to: 4-1BB nanobody, CD47 nanobody, VEGF nanobody, HER2 nanobody, EGFR nanobody, HER3 nanobody, B7H3 nanobody, TIGIT nanobody, OX-40 nanobody, CD40 nanobody or PD-L1 nanobody.

A seventh aspect of the present invention provides use of the above anti-4-1BB nanobody or bispecific antibody in the preparation of a medicament for preventing and/or treating a cancer and detecting 4-1BB protein.

In the embodiments provided by the present invention, the cancer includes but is not limited to: gastric cancer, liver cancer, leukemia, kidney tumor, lung cancer, small intestine cancer, bone cancer, prostate cancer, colorectal cancer, breast cancer, large intestine cancer, prostate cancer, cervical cancer, lymphoma, adrenal tumor or bladder tumor.

A eighth aspect of the present invention provides a pharmaceutical composition, which comprises: the aboved anti-4-1BB nanobody or bispecific antibody; and a pharmaceutically acceptable excipient.

In the embodiments provided by the present invention, the dosage forms of the pharmaceutical composition include but are not limited to: injection, powder injection, tablet or capsule.

A ninth aspect of the present invention provides a kit for detecting 4-1 BB protein, wherein the kit comprises: the above anti-4-1BB nanobody or bispecific antibody; and a reagent acceptable in detection.

### BRIEF DESCRIPTION OF THE DRAWINGS

Figure 1 shows the FACS binding ability of VHH-hFc recombinant antibody to 293F cells overexpressing human 4-1BB;
FIG2 shows the IL-2 release of PBMCs stimulated by VHH-hFc recombinant antibody (in the presence of OKT3);
FIG3 shows the IL-2 release of PBMCs stimulated by VHH-hFc recombinant antibody (in the absence of OKT3).

### DETAILED DESCRIPTION

The invention discloses an anti-4-1BB nanobody and a biological material and product thereof. A person skilled in the art can learn from the content of the application and appropriately improve the process parameters for implementation. It should be noted that, all similar substitutions and modifications are obvious to those skilled in the art, and they are deemed to be included in the present invention. The methods and applications of the present invention have been described through preferred embodiments. Relevant persons can obviously make modifications or appropriate changes and combinations to the methods and applications described herein without departing from the content, spirit and scope of the present invention to achieve and apply the technology of this invention.

Terminology explanations:
Nanobody (Nb), an antibody that naturally lacks light chains, exists in the peripheral blood of alpacas. This antibody only comprises one heavy chain variable region (VHH) and two conventional CH2 and CH3 regions, but does not like artificially modified single-chain antibody fragments (scFv), they easily stick to each other and even aggregate into clumps. More importantly, the separately cloned and expressed VHH structure has structural stability and antigen-binding activity comparable to that of the original heavy chain antibody, and is the smallest known unit that can bind the target antigen.

The framework region (FR) is the skeleton region within the variable region of an antibody. The sequences of about 110 amino acids near the N-terminus of the H and L chains of immunoglobulins vary greatly, while the amino acid sequences of the other parts are relatively constant. Based on this, the light chain and heavy chain can be divided into the variable region (V) and the constant region (C). The variable region comprises the hypervariable region (HVR) or complementary determining region (CDR, complementarity-determining region) and the FR skeleton region. The variability of FR is lower than that of CDR. There are four FR molecules, namely FR1, FR2, FR3 and FR4. When recognizing antibodies, the four FR molecules curl up to bring the CDR molecules closer to each other.

Complementarity determining region (CDR): the entire antibody molecule can be divided into two parts: the constant region and the variable region. In the variable region, there is a small part of amino acid residues that change particularly strongly. The residue composition and arrangement order of these amino acids are more prone to variation, which is called the hypervariable region. There are three hypervariable regions (HVR) in the V region of the L chain and H chain. Because this part can form precise complementarity with the antigenic determinant in terms of spatial structure, the hypervariable region is also called the complementarity determining region.

Bispecific antibody: an artificial antibody comprises two specific antigen-binding sites, which can build a bridge between a target cell and a functional molecule (cell) to stimulate a directed immune response. It is a type of genetically engineered antibody, and has become a hot topic in the field of antibody engineering. It has broad application prospects in tumor immunotherapy.

The names and abbreviations of the 20 kinds of amino acids:

| Three-letter Abbreviation | Abbreviation | English Name |
|---|---|---|
| Gly | G | Glycine |
| Ala | A | Alanine |
| Val | V | Valine |
| Leu | L | Leucine |
| Ile | I | Isoleucine |
| Phe | F | Phenylalanine |
| Trp | W | Tryptophan |
| Tyr | Y | Tyrosine |
| Asp | D | Aspartic acid |
| Asn | N | Asparagine |
| Glu | E | Glutamic acid |
| Lys | K | Lysine |
| Gln | Q | Glutamine |
| Met | M | Methionine |
| Ser | S | Serine |
| Thr | T | Threonine |
| Cys | C | Cysteine |
| Pro | P | Proline |
| His | H | Histidine |
| Arg | R | Arginine |

The reagents, instruments, bacterial strains or biological materials used in the present invention can all be obtained through commercial purchases.

The present invention will be further described below in conjunction with the following Examples:

### Example 1: Antigen Preparation and Animal Immunization

### 1. Animal immunity:

Human 4-1BB extracellular domain recombinant protein (hFc tag) was injected subcutaneously and intramuscularly at multiple points on the back of the neck of two alpacas to form multiple masses, and the absorption of the subcutaneously injected masses was tracked and observed to confirm correct immunization.

The first immunization:0.5 mg of antigen was used to mix with Freund's complete adjuvant at a ratio of 1:1, emulsifying and then injecting with a volume of 1 mL/alpaca;
The second immunization: 3 weeks after the first immunization, 0.25mg of antigen was used to mix with Freund's incomplete adjuvant at a ratio of 1:1, emulsifying and then injecting with a volume of 1 mL/alpaca;
The third immunization: 3 weeks after the second immunization, 0.25 mg of antigen was used to mix with Freund's incomplete adjuvant at a ratio of 1:1, emulsifying and then injecting with a volume of 1 ml/alpaca;
The fourth immunization: 3 weeks after the third immunization, 0.25mg of antigen was used to mix with Freund's incomplete adjuvant at a ratio of 1:1, emulsifying and then injecting with an volume of 1mL/alpaca.

### 2. Serum processing and titer detection:

One week after the third and fourth immunizations, 2 mL of peripheral blood was collected, and serum was separated. The human 4-1BB extracellular domain recombinant protein (his-tag) was coated in an ELISA 96-well plate, and the antibody titer in the serum was determined by ELISA. The ELISA results show that the titers of the four-immune serum of the two alpacas are >1:32000, which meet the library construction standards.

### Example 2: Construction of Phage Display Immune Antibody Library

Since the serum titers of the alpacas after the fourth immunization are>1:32000, it indicates that there are high-affinity antibodies against human 4-1BB in the serum. Therefore, we constructed a phage display immune antibody library according to the following steps:
① 50 mL of peripheral blood from each of the two alpacas after the fourth immunization was collected to separate the lymphocytes PBMCs, and combine the PBMCs of the two alpacas; 2×10⁷ PBMCsare taken to extract total RNA by using an RNA extraction kit; an appropriate amount of RNA (such as 3-5 µg)was taken to obtain cDNA by using an RT-PCR reverse transcription kit.
②The IgG2 and IgG3 heavy chain variable region sequences (the heavy chain variable region VHH of the nanobody) were obtained step by step by nested PCR. The experimental steps are as follows: 1) a pair of specific nested outer primers were designed for the first round of PCR amplification by using cDNA as a template. The amplified region is the Leader-CH2 region of the alpaca heavy chain antibody gene, and the product size is 700bp and 900bp; DNA gel electrophoresis and gel cutting were used to recover the 700bp PCR product; 2) nested inner primers (3 pairs for alpaca) were designed for the second round of PCR amplification by using the 700bp first round PCR product as a template. The amplified region is the VHH fragment of the alpaca heavy chain antibody variable region, and the product size is 400bp; a PCR product purification kit was used to purify and recover the second round PCR product.
③ The heavy chain variable region sequence was inserted into the linearized phagemid vector VHH-lib Template treated with enzyme digestion by homologous recombination or enzyme ligation, to obtain the recombinant vector; after purification and recovery, the super competent SS320 cells (containing the helper phage M13K07) were transformed; the transformed bacterial solution was resuspended in SOC medium to activate it for 1 hour; a small amount of bacterial solution was taken for 10-fold gradient dilution, an appropriate dilution titer was selected, spreading on LB/tet10 and LB/Carb50 culture plates, placing in a 37°C biochemical incubator overnight, and then using it for library capacity calculation the next day; the remaining bacterial solution was transferred to a large volume of 2YT/Carb₅₀/Kan₂₅ liquid culture medium, placing in a 37°C shaker to culture overnight, harvesting the supernatant the next day, 1/4 volume of PEG/NaCl solution was added to precipitate the phage, an appropriate amount of PBT solution was taken to resuspend the phage and dilute to the required concentration to obtain the phage display immune antibody library (stored at -80°C for future use).
④The number of clones on the LB/Carb₅₀ plate was counted, and the library capacity was calculated: the library capacity of the alpaca antibody library Lib 4-1BB Alpaca is 4.60×10⁹. 20 monoclonal sequences were randomly selected from each plate, and the results show that the VHH insertion efficiency of the alpaca antibody library Lib 4-1BB Alpaca is 90%.

### Example 3: Screening of Antibody Library

5µg/mL of human 4-1BB extracellular domain recombinant protein (his-tag) was added to a 96-well plate (100µL/well)to coat overnight at 4°C; NEB5αF' *Escherichiacoli* was streaked on a 2YT/Tet₁₀ plate to culture overnight in an incubator at 37°C; the next day, NEB5αF' monoclonal clone was picked from the overnight 2YT/Tet10 plate to add to 3mL of 2YT/Tet10 liquid culture medium, and the bacteria were grown at 37°C until OD600 = 0.8; at the same time, the antigen supernatant of the 96-well plate was removed, 200µL of 1% BSA was added to each well for blocking, and 200µL of 1% BSA was added to blank well as the negative control well, placing on a 3D rotating shaker at room temperature for 2 hours; then, the supernatant of the protein well and the control well was removed, washing with 200µLPT, and 100µL of phage antibody library was added to each well, placing on a 3D rotating shaker at room temperature for 2hr; the supernatant of the protein well and the control well was removed, washing with 200µL PT; 100µL of 100mM HCl was added to the well, placing at room temperature for 5 minutes; the supernatant was aspirated to add to a 1.5mL centrifuge tube, neutralizing with 1M Tris-HCl. The above mixture was added to a centrifuge tube containing 1 mL of NEB5αF' bacteria, shaking and culturing at 37°C for 1 hour; 20 µL of the culture solution in the centrifuge tube was taken to dilute to an appropriate multiple, spreading on an LB/Carb50 culture plate, placing in a 37°C biochemical incubator overnight, and using for calculation of titer and enrichment the next day; 1µL of helper phage M13K07 (final concentration of 1010 cells/mL) was added to the remaining culture solution, shaking at 37°C to culture for 1 hour; the above culture solution was transferred to 35mL of 2YT/Carb50/Kan25 culture solution, placing in a shaker to culture overnight at 37°C, and then phages were collected to form an antibody library for each round.

The above steps were repeated for 2-3 rounds until phage enrichment occurs. If the number of colonies in the antigen-binding wells on the LB/Carb50 plate is more than 10 times that of the negative control wells, the enrichment is considered successful. In this experiment, after the second round of screening, the number of colonies in the antigen-binding wells of Lib 4-1BB Alpaca was 100 times that of the negative control wells, indicating successful enrichment.

Clones from the enrichment round were randomly picked to expand and culture in 96-deep-well plates, and after centrifugation the supernatant was used for Phage ELISA screening. Clones having a ratio of OD values while binding to human 4-1BB extracellular domain recombinant protein (his-tag): OD values greater than 2 while binding to blocking solution were selected to defined as positive clones, which were sequenced and aligned to obtain unique sequences.

### Example 4: NGS Sequencing

Designed primers were located in the constant regions on both sides of VHH, the variable region of the phage library was amplified, E-gel detection was performed on the PCR products, and then the kit was used to purify and recover the PCR library fragments for NGS sequencing. The obtained data were statistically analyzed by using SPSS 2.0 and Microsoft Excel 2019, and sorted according to the number and frequency of DNA.

### Example 5: Alignment and Statistics of Positive Sequences and NGS High-frequency Sequences

The phage ELISA positive sequences and NGS high frequency sequences (Top 50) were compared and grouped according to the differences in CDR H3. The results are shown in Table 1.

**Table 1. 4-1BB nanobodies obtained from alpaca immunization**

| **Group** | **Unique identifier** | **VHH amino acid sequence** | **Source** |
|---|---|---|---|
| 1 | 137-1 | QVQLVESGGGLVQPGGSLRLSCVASGF VFSTVTMGWGRQVPGKEFEWVSGVSSE GITDYADSVKGRFTISRDNAKNTVYLEM NSLKSEDTAEYYCLIGGRWSFGTWGQG TQVTVSS | Phage ELISA Positive |
| 2 | 137-7 | QVQLVESGGGLVQPGGSLRLSCAASGF TLDSYAIAWFRQAPGKEREGVSCITSSGG STNYADSVKGRFTISRDGAKNTVYLQLN SLKPEDTAIYYCAAFHRATCVILGEYDY WGQGTQVTVSS | NGS Top7 |
| 3 | 137-12 | QVQLVESGGGLVHPGGSLRLSCAASGF TLDDSAIGWFRQAPGKEREGVSCRSRSG YMTKYADSVKGRFTISRYNANNTMYLQ MNSLKPEDTAVYYCATTRRGYCSGYET KGVDYWGQGIQVTVSS | NGS Top12 |
| 4 | 137-16 | EVQLVESGGGLVQAGASLTLSCVISGR TFSSYAMGWYRQAPGKERAFVGTITWN NDIIYSDSVKGRFTISTDDGKNTMYLQM NNLKPEDTAVYYCAAYSVPYGDFCTLN YDLMDYWGKGTLVTVSS | NGS Top16 |
| 5 | 137-18 | QVQLVESGGGLVHPGGSLRLSCAASGF IFSLYTMSWVRQAPGKGLEWVSDINSGG DSTDYADSVKGRFTISRDNAKSTLYLQM NSLKPEDTAVYYCAKDYPLGQREDYW GQGTQVTVSS | NGS Top18 |
| 6 | 137-36 | QVQLVESGGGLVQTGGSLTLSCVASGR RFEAYTIGWLRQAPGDLREGVGCIFGGT TFTGYDDSVKGRFTISRDTAKNTVYLRM NNLTSEDTATYYCAAEGRPGQIKGQHG CLTPSWYNYKGQGTQVTVSS | NGS Top36 |
| 7 | 137-39 | QVQLVESGGGLVHVGESLTLSCVISGG SSNLYGIGWYRQAPGQERKLVAVLHNE HTTKYLDSVKGRFFVSGDNAKNTLYLH MNNVKVEDTAVYYCNSASGTLAGTGYF ASVGPWGQGTLVTVSS | NGS Top39 |

| | | | |
|---|---|---|---|
| Note: The underlined sequences are CDRs | | | |

### Example 6: Eukaryotic Expression of Nanobodies

A total of 7 sequences in Table 1 of Example 5 were eukaryotic expressed. The experimental steps were: 1) the VHH fragments of these sequences were amplified by PCR, and the fragments were inserted into the eukaryotic expression vector pFclG comprising human IgG1 Fc tag; the vector was electrotransfected into *E.coli* trans5α host strain, then screening with bleomycin, and conducting single clone sequencing to obtain the correct recombinant plasmid; then the host strain comprising the recombinant plasmid was expanded and cultured, sterile endotoxin-free plasmids were obtained by endotoxin-free kit; 2) serum-free medium was used to culture HEK293F cells; Polyplus suspension cell transfection reagent was used to transfer the recombinant expression plasmid into HEK293F cells for expression. Supplementary materials were added 24 and 72 hours after transfection, and the supernatant was collected on day 5. Antibodies were separated and purified by using Protein A agarose purification resin, and the antibodies were replaced and stored in PBS. The experimental results (Table 2) show that the yields of transient expression of VHH-hFc recombinant antibodies of the 7 sequences in 293F cells are 53-231.4mg/L; SDS-PAGE electrophoresis identification: the VHH-hFc band sizes of 137-1, 137-7, 137-12, 137-18, and 137-36 sequences are normal, and the purity is >95%; the recombinantly expressed antibodies of 137-16, and 137-39 sequences have non-specific bands, and the purity is <95%.

**Table 2. Eukaryotic transient expression results of VHH-His recombinant antibodies**

| **Unique identifier** | **Antibody type** | **Expression volume(mL)** | **Production(mg)** | **SDS-PAGE Purity** |
|---|---|---|---|---|
| 137-1 | VHH-hFc | 50 | 6.94mg | >95% |
| 137-7 | VHH-hFc | 50 | 7.08mg | >95% |
| 137-12 | VHH-hFc | 50 | 5.72mg | >95% |
| 137-16 | VHH-hFc | 50 | 2.83mg | >70% |
| 137-18 | VHH-hFc | 50 | 4.79mg | >95% |
| 137-36 | VHH-hFc | 50 | 5.73mg | >95% |
| 137-39 | VHH-hFc | 50 | 1.59mg | About 30% |

### Example 7: Affinity EC50 of Nanobodies and Antigen Recombinant Proteins

The affinity of the VHH-hFc recombinant antibodies of the seven sequences to the recombinant proteins of the extracellular region of human and monkey 4-1BB was determined by ELISA method: the recombinant proteins of the extracellular region of human and monkey 4-1BB (his-tag) were added to the ELISA 96-well plate, 200ng/well, to coat overnight at 4°C. The VHH-hFc recombinant antibodies were diluted to different concentrations (0.014-10µg/mL)to respectively react with the antigen by ELISA, and the HRP-labeled anti-VHH secondary antibody was used for color development, and the absorbance value at 450 nm was measured by ELISA instrument. The experimental results (Tables 3 and 4) show that, the VHH-hFc recombinant antibodies of the seven sequences can bind to the recombinant proteins of the extracellular region of human or monkey 4-1BB, wherein the affinity of CD137-1, CD137-7, CD137-16, and CD137-18 to the recombinant proteins of the extracellular region of human or monkey 4-1BB ranks in the top four. The same experimental method was used to detect the affinity of the recombinant antibodies to the mouse 4-1BB extracellular region recombinant protein. The results (Table 5) show that, the VHH-hFc recombinant antibodies of CD137-1, CD137-7, CD137-16, and CD137-18do not bind or weakly bind to the mouse 4-1BB extracellular region recombinant protein.

**Table 3. ELISA binding capacity of VHH-hFc recombinant antibody and human 4-1BB (his-tag)**

| **Human4-1BB, his-tag (200ng/well coating)** | | | | | | | |
|---|---|---|---|---|---|---|---|
| Concentration(µg/ml) | 137-1 | 137-7 | 137-12 | 137-16 | 137-18 | 137-36 | 137-39 |
| 10.0 | OVER | OVER | 0.432 | OVER | 2.930 | 0.480 | 1.451 |
| 3.33 | OVER | OVER | 0.433 | OVER | 1.734 | 0.454 | 1.003 |
| 1.11 | OVER | OVER | 0.376 | 3.517 | 1.308 | 0.414 | 0.620 |
| 0.37 | 3.970 | OVER | 0.341 | 1.577 | 0.732 | 0.347 | 0.458 |
| 0.12 | 1.498 | 2.372 | 0.334 | 0.704 | 0.562 | 0.383 | 0.461 |
| 0.041 | 0.754 | 0.963 | 0.355 | 0.419 | 0.451 | 0.372 | 0.477 |
| 0.014 | 0.612 | 0.589 | 0.395 | 0.410 | 0.399 | 0.413 | 0.521 |
| 0 | 0.093 | 0.081 | 0.087 | 0.096 | 0.119 | 0.112 | 0.128 |
| EC50(µg/ml) | 0.138 | 0.090 | 6.25E-05 | 0.536 | 4.061 | 0.001 | 6.045 |
| EC50(nM) | 1.726 | 1.120 | 0.001 | 6.703 | 50.763 | 0.011 | 75.563 |

**Table 4. ELISA binding ability of VHH-hFc recombinant antibody to monkey 4-1BB (his-tag)**

| **Monkey4-1BB, his-tag (200ng/well coating)** | | | | | | | |
|---|---|---|---|---|---|---|---|
| Concentration(µg/ml) | 137-1 | 137-7 | 137-12 | 137-16 | 137-18 | 137-36 | 137-39 |
| 10.0 | OVER | OVER | 1.074 | OVER | 3.303 | 1.913 | 0.405 |
| 3.33 | OVER | OVER | 0.679 | OVER | 2.053 | 0.879 | 0.342 |
| 1.11 | OVER | OVER | 0.423 | OVER | 1.194 | 0.456 | 0.286 |
| 0.37 | 3.972 | 3.189 | 0.343 | 2.787 | 0.707 | 0.276 | 0.251 |
| 0.12 | 1.847 | 1.539 | 0.376 | 1.169 | 0.433 | 0.372 | 0.306 |
| 0.041 | 0.971 | 0.794 | 0.370 | 0.581 | 0.392 | 0.354 | 0.387 |
| 0.014 | 0.621 | 0.581 | 0.385 | 0.447 | 0.325 | 0.333 | 0.292 |
| 0 | 0.077 | 0.055 | 0.058 | 0.043 | 0.051 | 0.041 | 0.041 |
| EC50(µg/ml) | 0.113 | 0.170 | 10.280 | 0.234 | 4.356 | 51.910 | 0.001 |
| EC50(nM) | 1.409 | 2.120 | 128.500 | 2.928 | 54.450 | 648.875 | 0.012 |

**Table 5. ELISA binding of VHH-hFc recombinant antibodies to mouse 4-1BB (his-tag)**

| **Mouse4-1BB, his-tag (200ng/wellcoating)** | | | | |
|---|---|---|---|---|
| Concentration (µg/ml) | CD137-1 | CD137-7 | CD137-16 | CD137-18 |
| 10.0 | 0.31 | 0.284 | 0.816 | 0.288 |
| 3.33 | 0.231 | 0.218 | 0.308 | 0.219 |
| 1.11 | 0.207 | 0.191 | 0.213 | 0.214 |
| 0.37 | 0.269 | 0.201 | 0.197 | 0.199 |
| 0.12 | 0.184 | 0.166 | 0.184 | 0.192 |
| 0.041 | 0.202 | 0.18 | 0.183 | 0.182 |
| 0.014 | 0.199 | 0.187 | 0.188 | 0.188 |
| 0 | 0.24 | 0.209 | 0.218 | 0.203 |

### Example 8: Affinity KD Values of Nanobody and Antigen Recombinant Protein

Biacore was used to detect the affinity KD values of the VHH-hFc recombinant antibody of the 4 sequences (CD137-1, CD137-7, CD137-16, and CD137-18)to the human 4-1BB extracellular region recombinant protein: Biacore 8k setup sample bin, and the flow chamber temperature is 25°C. The Protein A chip captures the VHH-hFc sample as Ligand, and the corresponding target protein is diluted in HBS-EP+pH7.4 buffer as Analytes. The Analyte flow rate is 30 µL/min, binding for 120s, and dissociation for 600s. The data is analyzed by using 1:1 binding and Fit local Model fitting. The experimental results (Table 6) show that, the VHH-hFc recombinant antibodies of the four sequences can bind to the human 4-1BB extracellular region recombinant protein, with KD values ranging from 3.3-38nM.

**Table 6. SPR binding affinity of VHH-hFc recombinant antibodies to human 4-1BB (his-tag)**

| **Sample** | **Kinetics Chi² (RU²)** | **Ka(1/Ms)** | **Kd(1/s)** | **KD(M)** |
|---|---|---|---|---|
| CD137-1 | 3.25E-01 | 2.34E+06 | 9.16E-03 | 3.91E-09 |
| CD137-18 | 1.28E-01 | 2.36E+05 | 7.92E-04 | 3.36E-09 |
| CD137-7 | 3.37E-03 | 7.83E+05 | 2.98E-02 | 3.80E-08 |
| CD137-16 | 8.16E-03 | 8.22E+04 | 2.74E-03 | 3.33E-08 |

### Example 9: Binding of Nanobodies to Cells Overexpressing Antigen Recombinant Proteins

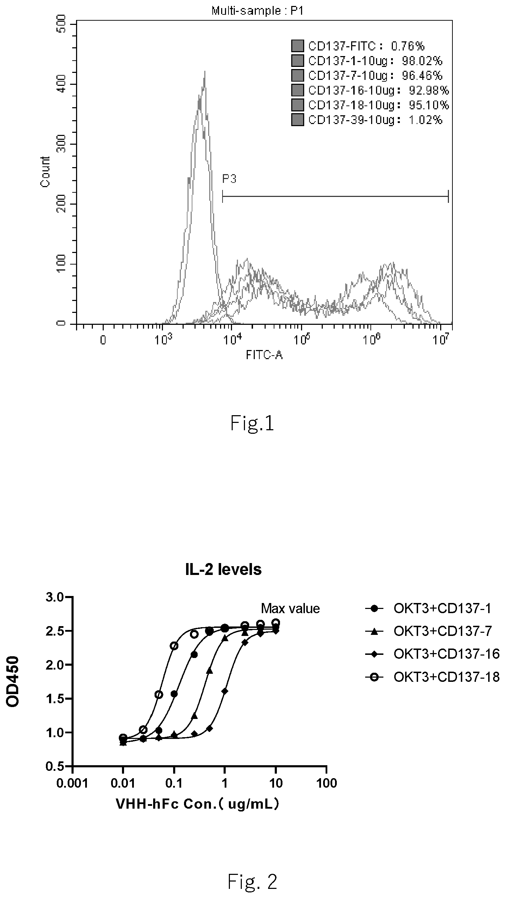

The affinity of the nanobody to human 4-1BB-overexpressing 293F cells was detected by flow cytometry: 1) 293F cells were transfected with a eukaryotic expression plasmid comprising the full-length 4-1BB geneto culture for 24 hours; 2) 0.3×10⁶ 293F cells transiently overexpressing human 4-1BB were taken to wash twice with PBS, then resuspending with 100µL PBS; 3) the cells were then incubated with 10µg/mL VHH-hFc recombinant antibody for 1 hour; 4) After washing the cells 3 times with PBS, the cells were resuspended with 100µL PBS, adding 0.2µg/mL FITC-labeled Anti-human Fc antibody to incubate for 1 hour; 4) After washing the cells 3 times with PBS, the cells were resuspended with 300µL PBS, and the fluorescence was detected by flow cytometer. The experimental results (Figure 1) show that, the VHH-hFc recombinant antibodies of CD137-1, CD137-7, CD137-16, and CD137-18 can bind to 293F cells overexpressing human 4-1BB.

### Example 10: PBMC Activation Experiment

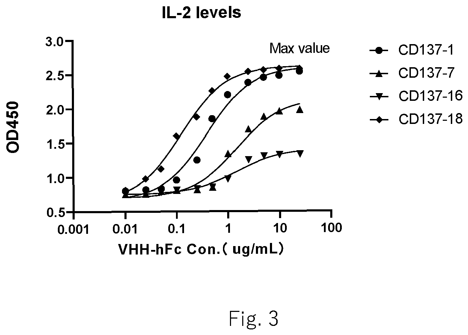

The ELISA kit was used to detect the level of IL-2 released after respectively stimulating human PMBC with VHH-hFc recombinant antibodies of the 4 sequences (CD137-1, CD137-7, CD137-16, and CD137-18): 96-well cell culture plates were coated with or without CD3 antibody OKT3, overnight at 4°C; PBMCs (1×10⁵/well) and different concentrations of 4-1BB recombinant antibodies were added the next day, and after 72 hours of continued culture, the supernatant was collected for ELISA detection of IL-2 levels. The experimental results (Figures 2 and 3) show that, VHH-hFc recombinant antibodies of CD137-1, CD137-7, CD137-16, and CD137-18 can stimulate human PBMCs to release IL-2 when coated with OKT3. However, in the absence of OKT3, the ability of CD137-1, CD137-7, CD137-16, and CD137-18 to activate PBMCs to release IL-2 was significantly reduced, and the EC50 value of the antibody was significantly increased (Table 7). In addition, the experimental results (Figure 3) show that, the values of maximum release of IL-2 (Max values) after stimulating PBMCs with CD137-1 and CD137-18in the presence or absence of OKT3are close, indicating that CD137-1 and CD137-18 VHH-hFc recombinant antibodies can 100% activate PBMCs with or without OKT3. However, CD137-7 and CD137-16 VHH-hFc recombinant antibodies cannot 100% activate PBMCs in the absence of OKT3, and the values of maximum release of IL-2 (Max value) of these two antibodies in the absence of OKT3are significantly lower than those in the presence of OKT3. These data indicate that, 100% activation of PBMCs by CD137-1 and CD137-18 is independent of CD3 antibody, whereas 100% activation of PBMCs by CD137-7 and CD137-16 is dependent on CD3 antibody.

**Table7. EC50 Values of IL-2 produced by PBMCs Stimulated with VHH-hFc Recombinant Antibodies**

| **Conditions** | **CD137-1** | **CD137-7** | **CD137-16** | **CD137-18** |
|---|---|---|---|---|
| EC50 in the presence of OKT3 (µg/mL) | 0.1260 | 0.4108 | 1.102 | 0.05779 |
| EC50 in the absence of OKT3 (µg/mL) | 0.3946 | 1.506 | 1.482 | 0.1258 |

The above are only the preferred embodiments of the present invention. It should be pointed out that, those skilled in the art can also make several improvements and modifications without departing from the principles of the present invention. These improvements and modifications can also be made, and should be regarded as falling in the protection scope of the present invention.

## Claims

1. An anti-4-1BB antibody, a variant thereof, or an antigen-binding fragment thereof, wherein the anti-4-1BB antibody comprises three complementary determining regions CDR1, CDR2 and CDR3 of VHHs respectively named as 137-7,
| **SEQ ID NO:** | **Unique Identifier** | **VHH amino acid sequence** |
|---|---|---|
| 2 | 137-7 | QVQLVESGGGLVQPGGSLRLSCAAS GFTLDSYAIAWFRQAPGKEREGVSCIT SSGGSTNYADSVKGRFTISRDGAKNTV YLQLNSLKPEDTAIYYCAAFHRATCV ILGEYDYWGQGTQVTVSS |
preferably, the three complementary determining regions CDR1, CDR2 and CDR3 of a heavy chain variable region (VHH) have amino acid sequence:
CDR 1 of SEQ ID NO: 11, CDR 2 of SEQ ID NO: 12, and CDR 3 of SEQ ID NO: 13.

2. The antibody, the variant thereof, or the antigen-binding fragment according to claim 1, wherein the variant is a humanized variant or a homology variant.

3. The antibody, the variant thereof, or the antigen-binding fragment thereof according to claim 2, wherein the variant has at least 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9% identity to the amino sequence of SEQ ID NO : 2.

4. The antibody, the variant thereof, or the antigen-binding fragment thereof according to claim 1, wherein the antibody is selected from camel Ig, Ig NAR, Fab fragment, Fab' fragment, F(ab)'₂ fragment, F(ab)'₃ fragment, Fv, scFv, bi-scFv, (scFv)₂, mini-antibody, double-chain antibody, three-chain antibody, four-chain antibody, disulfide-stabilized Fv protein and single domain antibody (sdAb, nanoantibody), camel antibody, bispecific antibody or trispecific antibody.

5. The antibody, the variant thereof, or the antigen-binding fragment thereof according to claim 2 wherein the antibody comprises amino acid sequence as set forth in SEQ ID NO: 2.

6. A fusion protein, wherein it comprises the antibody and a variant thereof, or an antigen-binding fragments thereof according to any one claims 1-5,
preferably, the fusion protein also comprises a tag sequence (such as Poly-His, Hemagglutinin, c-Myc, GST, Flag-tag, etc.) or an IgG1-Fc protein sequence, or an additional epitope (such as targeting 4-1BB or targeting other epitopes other than 4-1BB) or an additional antibody active fragment (such as an antibody or an antibody active fragment targeting other epitopes of 4-1BB or the same epitope, or a ligand capable of binding to 4-1BB).

7. An antibody-drug conjugate, wherein it comprises the antibody and a variant thereof, or an antigen-binding fragment thereof according to any one of claims 1-5.

8. An isolated polynucleotide expressing an antibody or an antigen-binding fragment thereof, wherein the polynucleotide is capable of expressing the antibody and a variant thereof, or an antigen-binding fragment thereof described in any one of claims 1-5; the polynucleotide is capable of expressing the fusion protein described in claim 6; the polynucleotide is capable of expressing the antibody-drug conjugate described in claim 7.

9. A vector, wherein it comprises the polynucleotide according to claim 8, preferably the vector is a plasmid vector.

10. A host cell, wherein it comprises the polynucleotide according to claim 8 or the vector according to claim 9, preferably the host cell is a eukaryotic cell.

11. A pharmaceutical composition, wherein it comprises: the antibody and a variant thereof, or an antigen-binding fragment thereof according to any one of claims 1-5, the fusion protein according to claim 6, the antibody-drug conjugate according to claim 7, and optionally a pharmaceutically acceptable carrier.

12. Use of the antibody and a variant thereof, or an antigen-binding fragment thereof according to any one of claims 1-5, the fusion protein according to claim 6, and the antibody-drug conjugate according to claim 7 in the preparation of a medicament for treating and/or preventing a disease associated with 4-1BB.

13. A method for detecting whether a sample comprises T cells highly expressing 4-1BB, wherein it comprises a step of contacting the sample with the antibody and a variant thereof, or an antigen-binding fragment thereof according to claim 1, the fusion protein according to claim 6, or the antibody-drug conjugate according to claim 7, optionally the detection may be for a diagnostic purpose or non-diagnostic purpose.

14. A kit for detecting 4-1BB protein, wherein the kit comprises: the antibody and a variant thereof, or an antigen-binding fragment thereof according to any one of claims 1-5, the fusion protein according to claim 6, or the antibody-drug conjugate according to claim 7; and a reagent acceptable in detection.
